# EUROPÄISCHE PATENTSCHRIFT

(11) **EP 0 521 170 B1**
(45) Veröffentlichungstag und Bekanntmachung des Hinweises auf die Patenterteilung: **06.04.1994**
(21) Anmeldenummer: 91109584.2
(22) Anmeldetag: 11.06.1991
(51) Int. Cl.: A61B 17/06

(54) **Nahtmaterialpackung**
Package for suture materials
Empaquetage pour matériaux de suture

(43) Veröffentlichungstag der Anmeldung: 07.01.1993
(73) Patentinhaber: B. Braun Holding AG, 6020 Emmenbrücke (CH)
(72) Erfinder: Odermatt, Erich, Dr., CH-8212 Neuhausen (CH); Sulzberger, Robert, CH-8212 Neuhausen (CH); Hofstetter, Ruedi, CH-8212 Neuhausen (CH)
(74) Vertreter: Selting, Günther, Dipl.-Ing.

(56) Entgegenhaltungen:
- DE-A- 1 935 364
- DE-A- 2 532 992
- FR-A- 2 374 016
- US-A- 4 120 395
- US-A- 4 249 656
- US-A- 4 284 194
- US-A- 4 491 218
- US-A- 4 496 045
- US-A- 4 533 041
- US-A- 4 615 435
- US-A- 4 884 681

## Beschreibung

Die Erfindung betrifft eine Nahtmaterialpackung für chirurgisches Nahtmaterial, wobei das Nahtmaterial in einer Faltkarte derart enthalten ist, daß es auf einfache Weise kontrolliert herausgezogen werden kann.

DE-A-25 32992 beschreibt eine Nahtmaterialpackung, bei der die Faltkarte aus drei langgestreckten nebeneinander angeordneten und durch längslaufende Falzlinien voneinander getrennten Platten besteht. Zwei dieser Platten bilden Halteplatten, die das Nahtmaterial umschließen und die an ihrer einen Längskante durch eine längslaufende Falzlinie verbunden sind, während die anderen Längskanten der übereinander gefalteten Halteplatten durch eine Einsteckverbindung verbunden sind. Zusätzlich zu den beiden Haltplatten ist eine Deckplatte vorgesehen, die die gleiche Länge hat wie die Halteplatten und die über die obere Halteplatte gefaltet wird. Die beiden Halteplatten bilden den an den stirnseitigen Enden offenen Aufnahmeraum für das Nahtmaterial, das aus dem Ende des Aufnahmeraums herausragt. Die Faltkarte ist in einer Folienhülle untergebracht, die eine Reißkerbe aufweist. Beim Aufziehen der Folienhülle wird ein Teil der Deckplatte abgerissen, wodurch das Ende des Nahtmaterials zum Ergreifen freigelegt wird. Nachteilig ist bei dieser Nahtmaterialpackung, daß das maschinelle Einlegen des Nahtmaterials und das Schließen der Faltkarte Schwierigkeiten bereiten. Das Nahtmaterial wird auf die mittlere der drei nebeneinander angeordneten Platten, nämlich die eine Halteplatte, aufgelegt, um danach die andere Halteplatte und schließlich die Deckplatte darüber falten zu können. Das Festhalten des schleifenförmig gewickelten Nahtmaterials während des Zuklappens der Faltkarte ist schwierig, weil die Platten beim Schließen das Festhalten des Nahtmaterials in definierter Position behindern. Ein weiterer Nachteil besteht darin, daß Nahtmaterial, das eine gewisse Steifigkeit hat, sich infolge seiner Eigenspannung strecken und aus den stirnseitigen Enden des Aufnahmeraums herausdringen kann. Ferner ist ein ordnungsgemäßes Herausziehen der Fäden nicht gewährleistet, weil diese am Ende des Aufnahmeraums nicht geführt sind.

Der Oberbegriff des Patentanspruchs 1 geht aus von einer Nahtmaterialpackung nach US-A-4 615 435. Diese Nahtmaterialpackung weist eine Basisplatte auf, die an jeder ihrer Seiten über eine Falzlinie mit einer Halteplatte verbunden ist. Das Nahtmaterial wird schleifenförmig auf der Basisplatte verlegt und anschließend werden die Halteplatten darübergeklappt, wobei sie sich gegenseitig überlappen. Die Halteplatten sind kürzer als die Basisplatte und sie bedecken nur einen Teil der Länge des schleifenförmig verlegten Nahtmaterials. Das schleifenförmige Wickeln des Nahtmaterials erfolgt dadurch, daß seitlich von der Basisplatte vier Stifte angeordnet werden, um die das Nahtmaterial bei geöffneten Halteplatten herumgewickelt wird. Diese Nahtmaterialpackung erlaubt nur ein kreuzförmiges Wickeln des Nahtmaterials über der Basisplatte und keine gleichmäßige Verteilung über die Fläche der Basisplatte. Beim Herausziehen des Nahtmaterials können durch das kreuzförmige Wickeln Knoten entstehen. Die mit der Basisplatte verbundenen Halteplatten überdecken zwar das Nahtmaterial, bewirken aber keinen Verschluß, so daß das Nahtmaterial nach dem Entfernen der Wickelstifte unbeabsichtigt freikommen kann.

Der Erfindung liegt die Aufgabe zugrunde, eine Nahtmaterialpackung zu schaffen, die das definierte Festhalten des Materials während des Einlegens ermöglicht und bei der das Nahtmaterial während des Schließens der Faltkarte bereits von dieser festgehalten wird.

Die Lösung dieser Aufgabe erfolgt erfindungsgemäß mit den im Patentanspruch 1 angegebenen Merkmalen.

Bei der erfindungsgemäßen Nahtmaterialpackung sind die Halteplatten, die das Nahtmaterial unmittelbar umschließen, kürzer als die Nahtmaterialschleife. Die Nahtmaterialschleife kann an beiden Enden festgehalten werden, während sie auf die eine Halteplatte aufgelegt wird und während die andere Halteplatte darübergefaltet und mit der Einsteckverbindung verriegelt wird. Auf diese Weise wird der Nahtmaterialvorrat zwischen den relativ kurzen Halteplatten fixiert, während die Faltkarte im übrigen noch geöffnet ist. Bis zum Schließen der Einsteckverbindung können die Enden des Nahtmamaterialvorrats von geeigneten Greifern festgehalten werden. Danach können die Greifer den Vorrat loslassen, da er dann von den Halteplatten festgehalten wird. Die Halteplatten werden über die Basisplatte gefaltet, deren Länge so groß ist, daß sie über die Enden des Nahtmaterials hinausragt, um dieses zu schützen.

An einem der Längsenden der Basisplatte befindet sich die erste Deckplatte, die also eine Verlängerung der Basisplatte in Längsrichtung bildet. Die Deckplatte wird über die das Nahtmaterial umschließenden Halteplatten gefaltet, so daß die Basisplatte und die erste Deckplatte den Schutzraum für das Nahtmaterial bilden, das Nahtmaterial jedoch nicht festhalten und fixieren. Die Fixierung des Nahtmaterials erfolgt durch die im Innern des Aufnahmeraums befindlichen Halteplatten.

Zweckmäßigerweise schließt sich an das andere Ende der Basisplatte mit einer zweiten quer verlaufenden Falzlinie eine zweite Deckplatte an. Diese zweite Deckplatte bedeckt die erste Deckplatte von der gegenüberliegenden Seite her. Das Fadenende, das aus dem von der Basisplatte und der ersten Deckplatte gebildeten Aufnahmeraum herausragt, wird von der zweiten Deckplatte bedeckt und dient als Schutz für die Fadenenden und die eventuell an die Fäden armierten Nadeln. Zum Freilegen dieses Fadenendes beim öffnen der Packung kann die zweite Deckplatte eine Aufreißlinie aufweisen, entlang der sie zerreißt, um die freien Fadenenden oder die Nadeln freizulegen.

Die das Nahtmaterial enthaltende Faltkarte ist in einer flachen Hülle, die längs der Seitenränder verschweißt ist, untergebracht und die aufgerissen werden kann. Das Aufreißen startet an einer Reißkerbe am Rand der Hülle. Beim Aufziehen der Hülle reißt ein Teil der zweiten Deckplatte entlang der Schwächungslinie ab.

Um das kontrollierte Aufreißen der Hülle entlang der Schwächungslinie der Faltkarte sicherzustellen, ist gemäß einer bevorzugten Weiterbildung der Erfindung die zweite Deckplatte teilweise durch eine umgefaltete Verstärkungsplatte verstärkt, die eine im wesentlichen deckungsgleich mit der Schwächungslinie verlaufende Führungskante aufweist. Die abreißbare Lasche der zweiten Deckplatte ist somit doppeltliegend und sie hat eine solche Steifigkeit, daß das Folienmaterial der Hülle entlang der Begrenzungslinie dieser Lasche definiert aufreißt.

Im folgenden wird unter Bezugnahme auf die Zeichnungen ein Ausführungsbeispiel der Erfindung näher erläutert.

Es zeigen:
- Fig. 1: eine Ansicht des Zuschnitts der Faltkarte,
- Fig. 2: den Zuschnitt von Figur 1 nach dem Einlegen des Nahtmaterials und dem Schließen der Einsteckverbindung der Halteplatten,
- Fig. 3: das Zusammenfalten der Faltkarte,
- Fig. 4: die Faltkarte im Endstadium des Zusammenfaltens,
- Fig. 5: das Einstecken der Faltkarte in die Hülle und
- Fig. 6: das Aufreißen der Hülle der Fadenenden zum Entnehmen des Fadens bzw. der Fäden.

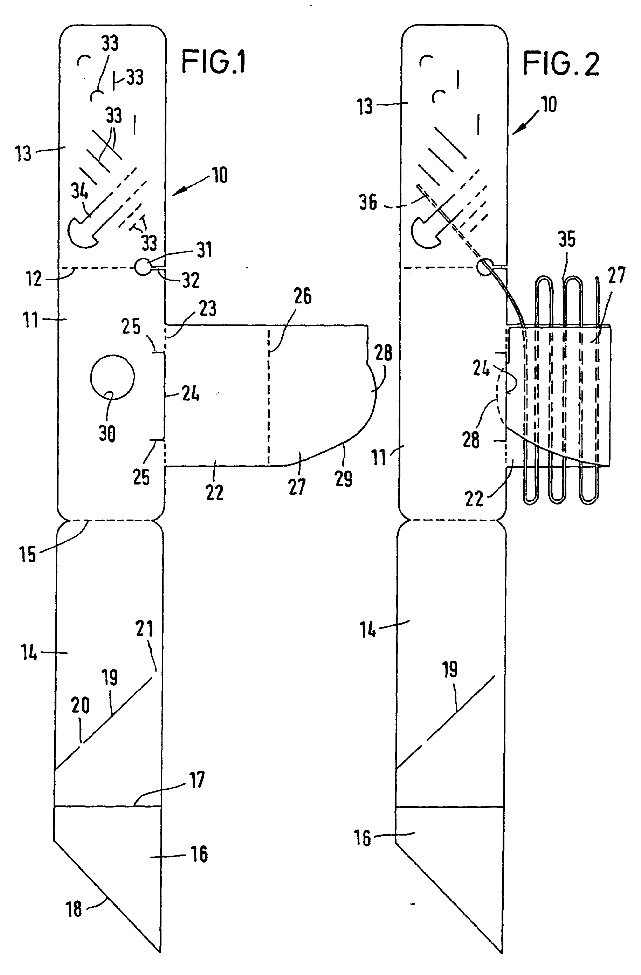

Gemäß Fig. 1 besteht die Faltkarte 10 aus einem einstückigen Zuschnitt aus Kartonmaterial oder flächigem Kunststoff. Die Faltkarte 10 weist eine langgestreckte rechteckige Basisplatte 11 auf, an deren eines Längsende sich über eine erste Falzlinie 12 eine erste Deckplatte 13 anschließt. Die erste Deckplatte 13 hat im wesentlichen die gleiche Form und Größe wie die Basisplatte 11, so daß sie über die Basisplatte gefaltet werden kann und diese dann im wesentlichen überdeckt. Die erste Deckplatte 13 ist allerdings geringfügig kürzer als die Basisplatte 11, so daß über sie die zweite Deckplatte 14 gefaltet werden kann, die sich an das entgegengesetzte Längsende der Basisplatte über eine zweite querlaufende Falzlinie 15 anschließt. Die Deckplatten 13 und 14 verlängern also die Basisplatte 11 in Längsrichtung, so daß alle drei Platten in Längsrichtung hintereinanderliegen und einen langgestreckten Streifen bilden.

An das Ende der zweiten Deckplatte 14 schließt sich eine etwa dreieckförmige Verstärkungsplatte 16 über eine dritte querlaufende Falzlinie 17 an. Die Verstärkungsplatte 16 hat an ihrem Ende eine schräg verlaufende Führungskante 18. Wenn die Verstärkungsplatte 16 um die Falzlinie 17 herumgefaltet wird, verläuft die Führungskante 18 kongruent mit einer in der zweiten Deckplatte 14 vorgesehenen schräg verlaufenden Schwächungslinie 19, die aus Schlitzen besteht, welche durch abreißbare Brücken 20,21 getrennt sind.

Von der Basisplatte 11 steht nach einer Seite hin eine erste Halteplatte 22 ab, die mit der Basisplatte über eine längslaufende Falzlinie 23 verbunden ist. In der Mitte der längslaufenden Falzlinie 23 befindet sich ein Schlitz 24, dessen Enden mit kurzen Einschnitten 25 der Basisplatte 11 verbunden sind.

Die Halteplatte 22 ist über eine längslaufende Falzlinie 26 mit der zweiten Halteplatte 27 verbunden, welche an ihrem äußeren Rand eine vorstehende Zunge 28 aufweist. Die beiden Halteplatten 22 und 27 haben etwa die gleiche Breite. Wenn die Halteplatte 27 um die Falzlinie 26 herumgefaltet wird, kann die Zunge 28 in den Schlitz 24 eingesteckt werden, so daß die Halteplatten im gegenseitigen Haltezustand verriegelt werden.

Die Längen der Halteplatten 22 und 27 sind kürzer als die Länge der Basisplatte 11. Genauer gesagt, ist die Länge der Halteplatten etwas größer als die halbe Länge der Basisplatte. Die Breite der Halteplatten ist minimal kleiner als die Breite der Basisplatte 11.

Obwohl die Halteplatten 22 und 27 im wesentlichen kongruent zueinander sind, hat die äußere Halteplatte 27 eine Abschrägung 29, die bewirkt, daß beide Halteplatten sich nicht vollständig überdecken und daß bei Bedarf die Einsteckverbindung leicht geöffnet werden kann.

In der Mitte der Basisplatte 11 befindet sich ein Loch 30 zur Ermöglichung der maschinellen Bewegung und Führung der Faltkarte 10.

Im Bereich der querlaufenden Falzlinie 12, die die erste Deckplatte 13 mit der Basisplatte 11 verbindet, ist ein Loch 31 vorgesehen, das sich in der Nähe des einen Seitenrandes befindet und für den Durchtritt des Fadenendes bestimmt ist. Von diesem Loch 31 führt ein Schlitz 32 bis zum Rand der Faltkarte. Dieser Schlitz 32 ermöglicht das seitliche Einführen des Fadenendes, mehrerer Fadenenden, auch solcher, die mit einer Nadel armiert sind, in das Loch 31.

Die erste Deckplatte weist mehrere verteilt angeordnete Schnittlinien 33 sowie eine Stanzlasche 34 auf, an denen das Fadenende bzw. die Fadenenden durch Einstecken oder Unterschieben fixiert werden kann. Beispielsweise kann eine am Fadenende befestigte Nadel in einen von den Schnittlinien 33 gebildeten Schlitz eingesteckt werden. Unter die Stanzlasche 34 können Fadenenden gesteckt werden.

Fig. 2 zeigt das in nebeneinanderliegenden Schleifen mäanderförmig auf der ersten Halteplatte 22 verlegte Nahtmaterial 35, über das die zweite Halteplatte 27 gefaltet wurde, bevor die Einsteckverbindung 24,28 geschlossen wurde. Das Nahtmaterial 35 ragt an beiden Enden über die Halteplatten 22,27 hinaus. Es wird während des Schließens der Halteplatten an einem Ende oder den Enden durch die Stanzlasche 34 oder die Schnittlinien 33 festgehalten. Nach dem Schließen der Halteplatten kann das Nahtmaterial losgelassen werden, weil es dann durch die Halteplatten, Stanzlaschen und Schnittlinien fixiert ist.

Das Nahtmaterial, das aus einem einzigen Faden oder aus mehreren Fäden besteht, weist ein abstehendes Fadenende 36 auf, das durch die Stanzlasche 34 oder die Schnittlinien 33 fixiert wird.

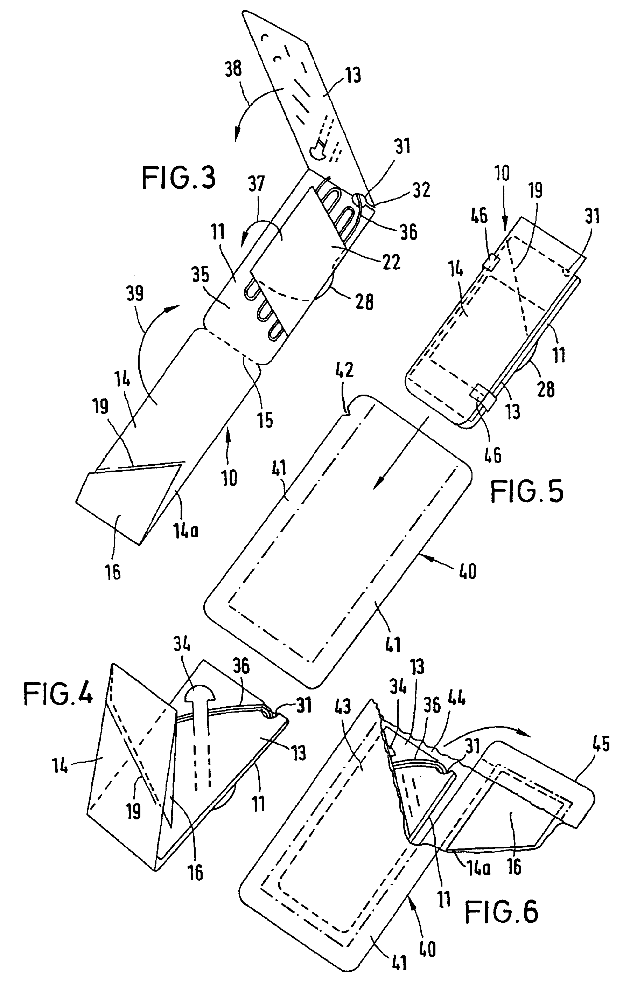

In Fig. 3 ist das Einfalten der beiden zusammenliegenden Halteplatten 22,27 auf die Basisplatte 11 in Richtung des Pfeiles 37, das anschließende Darüberfalten der ersten Deckplatte 13 in Richtung des Pfeiles 38 und das darauffolgende Darüberfalten der Deckplatte 14 in Richtung des Pfeiles 39 dargestellt. Ferner ist die Verstärkungsplatte 16 nach innen oder außen über die zweite Deckplatte 14 gefaltet. Das Fadenende 36 ist durch den Schlitz 32 seitlich in das Loch 31 eingeschoben.

Fig. 4 zeigt, daß mehrere Fadenenden 36 aus dem Loch 31 herausführen und unter der Stanzlasche 34 auf der Oberseite der ersten Deckplatte 13 festgelegt sind.

Gemäß Fig. 5 können über die Längsränder der zusammengefalteten Faltkarte 10 Klebelaschen 46 geklebt werden, um ein Auffalten zu vermeiden. Es ist zu erkennen, daß die zweite Deckplatte 14 die Basisplatte 11 und die erste Deckplatte 13 zu demjenigen Ende hin überragt, in dem sich das Loch 31 befindet.

Die Faltkarte 10 wird in die rechteckige Hülle 40 eingeschoben, die aus zwei entlang ihrer Ränder miteinander verschweißten Mehrschicht-Folien besteht. Die Schweißlinien sind mit 41 bezeichnet. Nach dem Einführen der Faltkarte 10 in die Hülle 40 erfolgt die Sterilisierung und anschließend wird die Einführöffnung ebenfalls verschweißt. An dem einen Längsrand der Hülle 40 ist eine Reißkerbe 42 vorgesehen. Diese befindet sich in Weiterführung der Schwächungslinie 19 der in die Hülle 40 eingeschobenen Faltkarte 10 dort, wo der Abstand von der zweiten querlaufenden Falzlinie 15 am größten ist.

Fig. 6 zeigt den Zustand des Aufreißens der Hülle, ausgehend von der Reißkerbe 42. Die vordere Folie 43 reißt entlang der Schwächungslinie 19 der an ihr anliegenden zweiten Deckplatte 14 auf, während die rückwärtige Folie 44 der Hülle 40 im wesentlichen entlang der querlaufenden Falzlinie 12 der Faltkarte aufreißt. Dabei wird die Lasche 14a zusammen mit der Verstärkungsplatte 16 von der zweiten Deckplatte 14 abgerissen und sie verbleibt in der Tasche der abgerissenen Hüllenlasche 45 auch ohne Ansiegelung an die Mehrschichtfolie. Dadurch werden die Fadenenden 36 auf der nunmehr freiliegenden Außenseite der Basisplatte 11 freigelegt. Diese Fadenenden können mit der Hand ergriffen werden, so daß der Faden bzw. die Fäden aus dem Loch 31 zusammen oder einzeln herausgezogen werden können.

Diese Einzelentnahme bei Mehrfadenpackungen ist durch die mäanderförmige Wickelung mehrerer Fäden auf der Halteplatte 22 und die Stabilisierung der Fadenschleifen durch die Halteplatte 27 möglich.

## Patentansprüche

1. Nahtmaterialpackung mit einer Faltkarte (10), die mehrere zusammenhängende übereinanderfaltbare Platten aufweist, wobei seitlich von einer Basisplatte (11) zwei Halteplatten (22,27) angeordnet sind, die kürzer sind als die Basisplatte und einen Teil der Länge des schleifenförmig gewickelten Nahtmaterials (35) bedecken, derart, daß das Nahtmaterial zu beiden Enden über sie hinaus vorsteht und wobei sich an das eine Längsende der Basisplatte (11) mit einer ersten querlaufenden Falzlinie (12) eine erste Deckplatte (13) anschließt, die die Basisplatte (11) im wesentlichen vollständig überdeckt,
**dadurch gekennzeichnet,**
daß nur die eine Halteplatte (22) mit der Basisplatte (11) über eine längslaufende Falzlinie (23) verbunden ist, und daß die andere Halteplatte (27) mit der einen Halteplatte (22) an ihrer einen Längskante durch eine Falzlinie (26) verbunden und an ihrer anderen Längskante mit einem ersten Teil (28) einer Einsteckverbindung (24,28) versehen ist, deren zweites Teil (24) an derjenigen Längskante der einen Halteplatte (22) vorgesehen ist, an der sich die längslaufende Falzlinie (23) befindet, wobei die beiden Halteplatten das Nahtmaterial unmittelbar zwischen sich einschließen.

2. Nahtmaterialpackung nach Anspruch 1, dadurch gekennzeichnet, daß sich an das andere Ende der Basisplatte (11) mit einer zweiten querlaufenden Falzlinie (15) eine zweite Deckplatte (14) anschließt.

3. Nahtmaterialpackung nach Anspruch 1 oder 2, dadurch gekennzeichnet, daß an der ersten querlaufenden Falzlinie (12) ein Loch (31) für den Durchtritt von Fadenenden (36) vorgesehen ist.

4. Nahtmaterialpackung nach Anspruch 3, dadurch gekennzeichnet, daß das Loch (31) zum einen Ende der der ersten querlaufenden Falzlinie (12) hin offen ist.

5. Nahtmaterialpackung nach einem der Ansprüche 1 bis 4, dadurch gekennzeichnet, daß die erste Deckplatte (13) Schnittlinien (33) und/oder Stanzlaschen (34) zur Festlegung von Fadenenden (36) oder Nadeln aufweist.

6. Nahtmaterialpackung nach einem der Ansprüche 1 bis 5, dadurch gekennzeichnet, daß die zweite Deckplatte (14) länger ist als die Basisplatte (11) und die erste Deckplatte (13).

7. Nahtmaterialpackung nach einem der Ansprüche 2 bis 6, dadurch gekennzeichnet, daß die zweite Deckplatte (14) über die erste Deckplatte (13) und die Basisplatte (11) hinaus in Längsrichtung übersteht und eine schräglaufende Schwächungslinie (19) aufweist und daß eine die Faltkarte (10) umschließende Hülle (40) eine Reißkerbe (42) an derjenigen Stelle hat, an der die Schwächungslinie (19) den größten Abstand von der zweiten querlaufenden Falzlinie (15) hat.

8. Nahtmaterialpackung nach Anspruch 7, dadurch gekennzeichnet, daß die zweite Deckplatte (14) teilweise durch eine umgefaltete Verstärkungsplatte (16) verstärkt ist, die eine parallel mit der Schwächungslinie (19) verlaufende Führungskante (18) aufweist.

9. Verfahren zum Einlegen von Nahtmaterial in eine Nahtmaterialpackung nach einem der Ansprüche 1 bis 8, dadurch gekennzeichnet, daß mindestens ein Fadenende an der Faltkarte (10) fixiert wird, daß Greifer den verbleibenden Fadenteil in Schleifen nebeneinander auf der der Basisplatte (11) benachbarten Halteplatte (22) anordnen, daß die andere Halteplatte (27) über das Nahtmaterial geklappt und verriegelt wird und daß die Greifer danach die über die Halteplatten hinausragenden Schleifenenden des Nahtmaterials loslassen.

## Claims

1. A suture material pack with a folding card (10) having a plurality of interconnected plates adapted to be folded over each other, wherein two holding plates (22,27) are arranged laterally of a base plate (11), the holding plates (22,27) being shorter than the base plate and covering a part of the length of the loop-shaped wound suture material (35) in such a manner that the suture material protrudes beyond the holding plates (22,27) at both ends, and wherein one longitudinal end of the base plate (11) is joined, via a first transversal crease line (12), with a first cover plate (13) substantially covering the whole base plate (11),
**characterized in**
that only the one holding plate (22) is connected to the base plate (11) through a longitudinal crease line (23) and that the other holding plate (27) has one of its longitudinal edges connected to said one holding plate (22) through a crease line (26) and has its other longitudinal edge provided with a first member (28) of a slip-in connection (24,28), of which the second member (24) is provided on that longitudinal edge of said one holding plate (22) which has the longitudinal crease line (23) arranged thereon, the two holding plates enclosing the suture material immediately between them.

2. The suture material pack according to claim 1, characterized in that the other end of the base plate (11) is joined by a second cover plate (14) via a second transversal crease line (15).

3. The suture material pack according to claim 1 or 2, characterized in that the a hole (31) for the passage of filament ends (36) is provided at the first transversal crease line (12).

4. The suture material pack according to claim 3, characterized in that the hole (31) is open towards one end of the first transversal crease line (12).

5. The suture material pack according to any one of claim 1 to 4, characterized in that the first cover plate (13) has cut lines (33) and/or punched flaps (34) for fixation of filament ends (36) or needles.

6. The suture material pack according to any one of claim 1 to 5, characterized in that the second cover plate (14) is longer than the base plate (11) and the first cover plate (13).

7. The suture material pack according to any one of claim 2 to 6, characterized in that the second cover plate (14) protrudes longitudinally beyond the first cover plate (13) and the base plate (11) and has an oblique weakened line (19), and that a sheath (40), enclosing the folding card (10), has a tear notch (42) at that location where the distance of the weakened line (19) from the second transversal crease line (15) is largest.

8. The suture material pack according to claim 7, characterized in that the second cover plate (14) is partly reinforced by a folded-over reinforcing plate (16) having a guide edge (18) extending in parallel to the weakened line (19).

9. A method for inserting suture material into a suture material pack according to any one of claims 1 to 8, characterized in that at least one filament end is fixed at the folding card (10), that gripping tools arrange the remaining filament portion side by side in loops on the holding plate (22) adjacent the base plate (11), that the other holding plate (27) is folded over the suture material and locked, and that the gripping tools thereafter release the loop ends of the suture material which protrude beyond the holding plates.

## Revendications

1. Emballage pour matériau de suture comportant une carte pliable (10), qui possède plusieurs plaques réunies pouvant être repliées les unes sur les autres, et dans lequel latéralement par rapport à une plaque de base (11) sont disposées deux plaques de retenue (22,27), qui sont plus courtes que la plaque de base et recouvrent une partie de la longueur du matériau de suture (35) enroulé sous la forme d'une boucle, de sorte que le matériau de suture fait saillie au-delà des deux extrémités de cette plaque, et dans lequel une première plaque de revêtement (13), qui recouvre sensiblement complètement la plaque de base (11), est raccordée à une extrémité longitudinale de la plaque de base (11) au moyen d'une première ligne transversale de pliage (12),
caractérisé en ce que
seule une plaque de retenue (22) est reliée à la plaque de base (11) par l'intermédiaire d'une ligne longitudinale de pliage (23), et en ce que l'autre plaque de retenue (27) est reliée à la première plaque de retenue (22) au niveau de l'un de ses bords longitudinaux par une ligne de pliage (26), et est munie, sur son autre bord longitudinal, d'une première partie (28) d'une liaison à enfichage (24,28), dont la seconde partie (24) est prévue sur le bord longitudinal de la première plaque de retenue (22), sur lequel est située la ligne de pliage longitudinale (23), les deux plaques de retenue enserrant directement entre elles le matériau de suture.

2. Emballage pour matériau de suture selon la revendication 1, caractérisé en ce qu'une seconde plaque de revêtement (14) se raccorde à l'autre extrémité de la plaque de base (11), par une seconde ligne transversale de pliage (15).

3. Emballage pour matériau de suture selon la revendication 1 ou 2, caractérisé en ce qu'un trou (31) pour le passage d'extrémités (36) d'un fil est prévu au niveau de la première ligne transversale de pliage (12).

4. Emballage pour matériau de suture selon la revendication 3, caractérisé en ce que la première ligne transversale de pliage (12) est ouverte.

5. Emballage pour matériau de suture selon l'une des revendications 1 à 4, caractérisé en ce que la première plaque de revêtement (13) comporte des lignes de découpe (33) et/ou des languettes de découpage (34) destinées à fixer les extrémités (36) d'un fil ou des aiguilles.

6. Emballage pour matériau de suture selon l'une des revendications 1 à 5, caractérisé en ce que la seconde plaque de revêtement (14) est plus longue que la plaque de base (11) et la première plaque de revêtement (13).

7. Emballage pour matériau de suture selon l'une des revendications 2 à 6, caractérisé en ce que la seconde plaque de revêtement (14) fait saillie dans la direction longitudinale au-delà de la première plaque de revêtement (13) et de la plaque de base (11) et possède une ligne oblique d'affaiblissement (19), et en ce qu'une enveloppe (40), qui enserre la carte pliable (10), possède une encoche d'arrachement (42) à l'emplacement où la ligne d'affaiblissement (19) est séparée de la seconde ligne transversale de pliage (15) par la distance maximale .

8. Emballage pour matériau de suture selon la revendication 7, caractérisé en ce que la seconde plaque de revêtement (14) est renforcée partiellement par une plaque de renforcement repliée (16), qui possède un bord de guidage (18) parallèle à la ligne d'affaiblissement (19).

9. Procédé pour l'insertion d'un matériau de suture dans un emballage pour matériau de suture selon l'une des revendications 1 à 8, caractérisé en ce qu'on fixe au moins une extrémité du fil sur la carte pliable (10), en ce que des organes de saisie disposent la partie restante du fil sous la forme de boucles côte à côte sur la plaque de retenue (22) voisine de la plaque de base (11), en ce qu'on rabat et on verrouille l'autre plaque de retenue (27) par-dessus le matériau de suture et en ce que les éléments de préhension libèrent ensuite les extrémités, qui font saillie au-delà des plaques de retenue, des boucles du matériau de suture.
